(19) **Europäisches Patentamt**

**European Patent Office**

**Office européen des brevets**

(11) Veröffentlichungsnummer: **0 424 775 B1**

(12) **EUROPÄISCHE PATENTSCHRIFT**

(45) Veröffentlichungstag der Patentschrift: **06.07.94**

(51) Int. Cl.⁵: **B23K 35/30**, C22C 5/02

(21) Anmeldenummer: **90119723.6**

(22) Anmeldetag: **15.10.90**

(54) **Lotlegierung für Dental- und Schmuckteile.**

(30) Priorität: **27.10.89 DE 3935813**

(43) Veröffentlichungstag der Anmeldung:
**02.05.91 Patentblatt 91/18**

(45) Bekanntmachung des Hinweises auf die
Patenterteilung:
**06.07.94 Patentblatt 94/27**

(84) Benannte Vertragsstaaten:
**AT BE CH DE DK ES FR GB GR IT LI LU NL SE**

(56) Entgegenhaltungen:
DE-A- 2 638 837
DE-A- 2 741 277
DE-A- 3 146 794
DE-B- 2 828 304
DE-C- 732 318

(73) Patentinhaber: **Degussa Aktiengesellschaft
Weissfrauenstrasse 9
D-60311 Frankturt(DE)**

(72) Erfinder: **Steinke, Rudi, Dipl.-Ing.
Grünaustrasse 7
W-6450 Hanau 9(DE)**
Erfinder: **Schittny, Stefan, Dr. Dipl.-Ing.
Rannenbergring 39
W-8755 Alzenau-Kälberau(DE)**
Erfinder: **Kempf, Bernd, Dr. Dipl.-Ing.
Am Trieb 28
W-6463 Freigericht(DE)**
Erfinder: **Groll, Werner, Dr. Dipl.-Ing.
Gartenstrasse 5
W-8755 Alzenau-Hörstein(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 424 775 B1

**Beschreibung**

Die Erfindung betrifft die Verwendung einer Lotlegierung für Dental-und Schmuckteile auf der Basis Gold-Palladium-Silber mit zusätzlichen Unedelmetallkomponenten.

Die Kostendämpfungsmaßnahmen im Gesundheitswesen in der BRD haben in den letzten Jahren zu einem verstärkten Einsatz von goldreduzierten Palladium-Basislegierungen in der Zahnmedizin geführt. Im Gegensatz zu vielen hochgoldhaltigen Legierungen weisen diese Legierungen eine weiße Farbe auf. Die bisher in der Zahntechnik verwendeten Lote sind von der Farbe her oft nicht optimal an diese neuen Legierungen angepaßt, da sie meist eine gelbe bzw. gelbliche Farbe besitzen.

Als Weißmacher von Goldlegierungen wird häufig Nickel verwendet. Es ist aber bekannt, daß nickelhaltige Legierungen beim Menschen zu Allergien führen können. In der Zahntechnik sollte daher nach Möglichkeit auf Nickel verzichtet werden. Gleiches gilt für weitere toxische Elemente, wie z.B. Cadmium, das Loten häufig zur Senkung des Schmelzbereiches zugefügt wird. Ein entsprechendes Lot ist beispielsweise in der deutschen Patentschrift 732 318 beschrieben.

Dentallotlegierungen müssen eine Reihe von Anforderungen erfüllen. Sie müssen mundbeständig sein, gutes Benetzungs- und Fließverhalten zeigen, in der Farbe und in ihrer Arbeitstemperatur sollen sie den zu verlötenden Legierungen angepaßt sein. Die Lotverbindung muß eine hohe Festigkeit aufweisen.

Für Dentalteile, die mit einer Keramikbeschichtung versehen werden, müssen die Lötverbindungen auch bei Brenntemperaturen von 950 bis 980° C eine ausreichende Festigkeit besitzen, damit es nicht zu Dimensionsänderungen bei gelöteten Teilen kommt. Da die Herstellung von metallischem Zahnersatz in mehreren Schritten vor sich geht, ist es zweckmäßig, neben einem Erstlot ein sogenanntes Zweitlot zur Verfügung zu haben, das in seiner Arbeitstemperatur soweit tiefer liegt, daß nachfolgende Lötungen durchgeführt werden können, ohne die zuerst gelötete Verbindung wieder zu lösen.

Im Bereich des Modellgusses mit Nichtedelmetall-Legierungen (z.B. Cobalt-Chrom-Legierungen) werden zum Löten häufig nickelhaltige Lote eingesetzt. Bei der Verbindung von Nichtedelmetall- und Edelmetall-Teilen wird das Unedelmetall zuerst mit einem nickelhaltigen Lot vorgeschwemmt und danach mit einem niedriger schmelzenden Lot gelötet. Da die Nichtedelmetall-Legierungen eine weiße Farbe aufweisen, ist auch in diesem Fall eine weiße Lotlegierung günstig. Eine Arbeitstemperatur von über 1000° C ist auch hier notwendig, wenn der Nichtedelmetall-Zahnersatz keramisch verblendet wird.

Zum Absenken des Schmelzbereiches gegenüber den im Prinzip ähnlich zusammengesetzten Dentallegierungen werden den Lotlegierungen niedrig schmelzende Elemente, wie Zink, Zinn, Indium, Gallium oder Germanium zulegiert. Zwei entsprechende Legierungstypen auf Gold-Basis sind in der deutschen Patentschrift 2 638 837 und in der US-Patentschrift 3 892 564 beschrieben. Beide Lottypen besitzen Arbeitstemperaturen im Bereich von 590 bis maximal 850° C. Sie sind daher für Teile, die einem Keramikbrand unterzogen werden, nicht brauchbar.

Auch im Bereich der Schmucklegierungen sind Lote gefragt, die in ihrer Farbe möglichst exakt mit den zu verlötenden Teilen übereinstimmen und hohe Festigkeiten der Lötverbindungen liefern. Da auch auf dem Schmucksektor nach Möglichkeit auf toxische und allergieauslösende Elemente verzichtet wird, ist es vorteilhaft, wenn auch das verwendete Lot keine entsprechenden Legierungsbestandteile aufweist. Gerade im Fall von nickelfreiem Weißgold ist es von Vorteil, auch ein weißes, nickelfreies Lot zur Verfügung zu haben.

Aus der DE-OS 27 41 277 sind Gußlegierungen für zahnärztliche Zwecke bekannt, die 40 bis 60 % Gold, 25 bis 40 % Silber, 5 bis 20 % Palladium, 1 bis 6 % Indium und 0,01 bis 0,1 % Germanium, Titan und/oder Cer-Mischmetall enthält. Außerdem können zur Härtesteigerung bis 6 % Zinn, bis 3 % Zink und bis zu je 1 % Eisen und/oder Kobalt zulegiert sein. Solche Gußlegierungen sind aber normalerweise nicht als Lote geeignet.

Es war daher Aufgabe der vorliegenden Erfindung, eine Lotlegierung für Dental- und Schmuckteile auf der Basis Gold-Palladium-Silber mit zusätzlichen Unedelmetallkomponenten zu entwickeln, die in ihrer Farbe den weißen Gold-Palladium- und Gold-Basislegierungen in der Dentaltechnik nahekommt, frei von gesundheitsbedenklichen Komponenten ist und deren Hochtemperaturfestigkeit Lötungen vor dem Keramikbrand gestattet.

Außerdem sollte sie bei Nichtedelmetall-Dentallegierungen und bei Schmuckteilen einsetzbar sein.

Diese Aufgabe wird erfindungsgemäß dadurch gelöst, daß eine Legierung verwendet wird, die aus 38 bis 70 Gew.-% Gold, 6 bis 20 Gew.-% Palladium, 14 bis 20 Gew.-% Silber, 1 bis 6 Gew.-% Eisen und/oder Kobalt, 0 bis 10 Gew.-% Kupfer, 0 bis 5 Gew.-% Zinn, 0 bis 5 Gew.-% Zink, 0 bis 5 Gew.-% Indium, 0 bis 4 Gew.-% Gallium, 0 bis 4 Gew.-% Germanium, 0 bis 1 Gew.-% Wolfram und 0 bis 1 Gew.-% Iridium, Ruthenium und/oder Rhenium besteht, wobei die Summe der Gehalte an Zinn, Zink, Indium, Gallium und Germanium mindestens 1 Gew.-% und höchstens 5 Gew.-% betragen darf.

Vorzugsweise verwendet man Legierungen mit 50 bis 70 Gew.-% Gold, 8 bis 20 Gew.-% Palladium, 14 bis 20 Gew.-% Silber, 1 bis 6 Gew.-% Eisen und/oder Kobalt, 1 bis 10 Gew.-% Kupfer, 0 bis 5 Gew.-% Zinn, 0 bis 5 Gew.-% Zink, 0 bis 5 Gew.-% Indium, 0 bis 4 Gew.-% Gallium, 0 bis 4 Gew.-% Germanium, 0 bis 1 Gew.-% Wolfram und 0 bis 1 Gew.-% Iridium, Ruthenium und/oder Rhenium, wobei die Summe der Gehalte an Zinn, Zink, Indium, Gallium und Germanium zwischen 1 und 5 Gew.-% liegen muß.

Bei Dental- und Schmuckteilen mit einem fast reinweißen Farbton, sollte man eine Lotlegierung verwenden, die neben den übrigen Bestandteilen 55 bis 70 Gew.-% Gold und 12 bis 16 Gew.-% Palladium enthält.

Der Zusatz von 1 bis 6 Gew.-% Eisen und/oder Cobalt zu Gold-Silber-Legierungen führt überraschenderweise zu einer Erhöhung der Zugfestigkeit dieser Legierungen bei Raumtemperatur und bei 900° C. Es werden dabei Zugfestigkeitswerte bei Raumtemperatur bis zu 500 MPa erreicht. Zusätzlich wirken sich Eisen und Kobalt durch ihre stark entfärbende Wirkung auch günstig auf die weiße Farbe der Legierung aus. Aus diesem Grund ist es möglich, den Anteil des Weißmachers Palladium relativ gering zu halten, da die Erhöhung des Palladium-Gehaltes zu einer unerwünschten Steigerung der Arbeitstemperatur führt. Für weiße Aufbrennlegierungen und weiße Nichtedelmetall-Legierungen sind daher Lotlegierungen mit 55 bis 70 Gew.-% Gold und 12 bis 16 Gew.-% Palladium besonders geeignet, da sie in ihrem Farbton den weißen Legierungen sehr ähnlich sind.

Die Elemente Zink, Zinn, Indium, Gallium und Germanium dienen zur Absenkung des Schmelzbereiches und zur Verbesserung der Fließfähigkeit. Ihr Gesamtanteil ist jedoch auf ca. 5 % in der Summe beschränkt, da ein größerer Anteil zu einer Versprödung des Materials führt, wodurch die Herstellung des Lotes in der gebräuchlichen Form als Band oder dünnes Stangenmaterial stark erschwert wird.

Über die Variation des Kupfer-Gehaltes läßt sich die Arbeitstemperatur in weitem Maße einstellen, ohne daß dadurch die Duktilität wesentlich beeinträchtigt wird. Die weiße Farbe der Lotlegierungen wird dabei fast nicht beeinflußt, wenn die Erhöhung des Kupfer-Gehaltes über eine Erniedrigung des Gold-Gehaltes kompensiert wird.

Der Zusatz von bis zu 1 Gew.-% Wolfram führt zu verbessertem Fließ- und Benetzverhalten der flüssigen Lote und damit zu zuverlässigeren Lötverbindungen. Iridium, Ruthenium und Rhenium können zur Kornfeinheitsverbesserung zulegiert werden.

Der Schmelzbereich, die Arbeitstemperatur und die Zugfestigkeit einiger erfindungsgemäßer Lotlegierungen wird in folgender Tabelle angegeben:

3

Tabelle

| Legierung | Au | Pd | Ag | Sonstige | Schmelzbereich [°C] | Arbeitstemperatur [°C] | Zugfestigkeit bei 20° C [ MPa ] |
|---|---|---|---|---|---|---|---|
| 1 | 66 | 14 | 14 | 2,8 Co 1,4 In 1,4 Zn 0,2 Ir | 1082 - 1141 | 1120 | 552 / 26 |
| 2 | 59,4 | 14 | 14 | 7,0 Cu 2,9 Fe 2,0 In 0,5 Wo 0,2 Ir | 995 - 1075 | 1070 | 522 / 25 |
| 3 | 58,4 | 8 | 20 | 7,0 Cu 2,0 Fe 3,0 In 1,0 Sn 0,5 W 0,1 Ir | 914 - 975 | 960 | 598 / n.b. |

**Patentansprüche**

1. Verwendung einer Legierung, bestehend aus 38 bis 70 Gew.-.% Gold, 6 bis 20 Gew. % Palladium, 14 bis 20 Gew.-% Silber, 1 bis 6 Gew.-% Eisen und/oder Kobalt, 0 bis 10 Gew.-% Kupfer, 0 bis 5 Gew.-% Zinn, 0 bis 5 Gew.-% Zink, 0 bis 5 Gew.-% Indium, 0 bis 4 Gew.-% Gallium, 0 bis 4 Gew.-% Germanium, 0 bis 1 Gew.-% Wolfram und 0 bis 1 Gew.-% Iridium, Ruthenium und/oder Rhenium, wobei die Summe der Gehalte an Zinn, Zink, Indium, Gallium und Germanium mindestens 1 Gew.-% und höchstens 5 Gew.-% betragen darf, als Lot für Dental- und Schmuckteile.

**2.** Verwendung einer Legierung, bestehend aus 50 bis 70 Gew.-% Gold, 8 bis 20 Gew.-% Palladium, 14 bis 20 Gew.-% Silber, 1 bis 6 Gew.-% Eisen und/oder Kobalt, 1 bis 10 Gew.-% Kupfer, 0 bis 5 Gew.-% Zinn, 0 bis 5 Gew.-% Zink, 0 bis 5 Gew.-% Indium, 0 bis 4 Gew.-% Gallium, 0 bis 4 Gew.-% Germanium, 0 bis 1 Gew.-% Wolfram und 0 bis 1 Gew.-% Iridium, Ruthenium und/oder Rhenium, wobei die Summe der Gehalte an Zinn, Zink, Indium, Gallium und Germanium zwischen 1 und 5 Gew.-% liegen muß, gemäß Anspruch 1.

## Claims

**1.** The use of an alloy, consisting of 38 to 70 % by weight gold, 6 to 20 % by weight palladium, 14 to 20 % by weight silver, 1 to 6 % by weight iron and/or cobalt, 0 to 10 % by weight copper, 0 to 5 % by weight tin, 0 to 5 % by weight zinc, 0 to 5 % by weight indium, 0 to 4 % by weight gallium, 0 to 4 % by weight germanium, 0 to 1 % by weight tungsten and 0 to 1 % by weight iridium, ruthenium and/or rhenium, where the sum of the contents of tin, zinc, indium, gallium and germanium is to amount to a minimum of 1 % by weight and a maximum of 5 % by weight, as solder for dental and jewellery parts.

**2.** The use of an alloy, consisting of 50 to 70 % by weight gold, 8 to 20 % by weight palladium, 14 to 20 % by weight silver, 1 to 6 % by weight iron and/or cobalt, 1 to 10 % by weight copper, 0 to 5 % by weight tin, 0 to 5 % by weight zinc, 0 to 5 % by weight indium, 0 to 4 % by weight gallium, 0 to 4 % by weight germanium, 0 to 1 % by weight tungsten and 0 to 1 % by weight iridium, ruthenium and/or rhenium, where the sum of the contents of tin, zinc, indium, gallium and germanium must amount to between 1 and 5 % by weight, in accordance with Claim 1.

## Revendications

**1.** Utilisation d'un alliage, constitué de 38 à 70 % en poids d'or, 6 à 20 % en poids de palladium, 14 à 20 % en poids d'argent, 1 à 6 % en poids de fer et/ou de cobalt, 0 à 10 % en poids de cuivre, 0 à 5 % en poids d'étain, 0 à 5 % en poids de zinc, 0 à 5 % en poids d'indium, 0 à 4 % en poids de gallium, 0 à 4 % en poids de germanium, 0 à 1 % en poids de tungstène et 0 à 1 % en poids d'iridium, de ruthénium et/ou de rhénium, la somme des teneurs en étain, zinc, indium, gallium et germanium devant présenter au moins 1 % en poids et au maximum 5 % en poids, en tant que brasure pour pièces dentaires et de bijouterie.

**2.** Utilisation d'un alliage, constitué de 50 à 70 % en poids d'or, 8 à 20 % en poids de palladium, 14 à 20 % en poids d'argent, 1 à 6 % en poids de fer et/ou de cobalt, 1 à 10 % en poids de cuivre, 0 à 5 % en poids d'étain, 0 à 5 % en poids de zinc, 0 à 5 % en poids d'indium, 0 à 4 % en poids de gallium, 0 à 4 % en poids de germanium, 0 à 1 % en poids de tungstène et 0 à 1 % en poids d'iridium, de ruthénium et/ou de rhénium, la somme des teneurs en étain, zinc, indium, gallium et germanium devant être comprise entre 1 et 5 % en poids, selon la revendication 1.